# EUROPEAN PATENT APPLICATION

(11) **EP 2 526 868 A1**
(43) Date of publication of application: **28.11.2012**
(21) Application number: 11305641.0
(22) Date of filing: 26.05.2011
(51) Int. Cl.: A61B 6/00

(54) **X-ray imaging apparatus having a variable distance between an X-ray source and an object to be imaged**

(71) Applicant: General Electric Company, Schenectady, NY 12345 (US)
(72) Inventor: Gorges, Sébastien, 78533 Buc (FR); Bismuth, Vincent, 78533 Buc (FR); Kotian, François, 78533 Buc (FR); Trousset, Yves, 78533 Buc (FR)
(74) Representative: Delprat, Olivier

(57) **Abstract**

This X-ray imaging apparatus comprises a rotary arm (5) and an image assembly (2) comprising an X-ray source (3) and an X-ray detector (4) so that the X-rays emitted by the X-ray source are incidental to the detector.
The X-ray source (3) is coupled to the rotary arm (5) through a movable support (9) able to set the distance between the X-ray source and the object and the movement of the X-ray source being dynamically controlled during a tridimensional imaging.

## Description

The present invention relates in general to medical imaging systems and, notably, to medical imaging systems for generating an image sequence for a tridimensional image reconstruction by means of an imaging assembly mounted on a rotary arm, moved on a circular part when an image sequence is generated.

X-ray imaging apparatuses for tridimensional (3D) image reconstruction usually comprise an imaging assembly having an X-ray source and an X-ray detector placed opposite to the X-ray tube in the direction of emission of the X-rays. The tube and the detector are placed on two mutually opposite ends of a rotary arm.

The rotary arm is a C-arm shaped in the form of an arch.

During examination, radiographs are produced by means of X-rays emitted by the X-ray source which irradiate a region of interest (ROI) of the body of the patient.

For this purpose, after the patient has been laid out on an examination table, the X-ray tube and the detector are brought to face the area to be radiographed.

For tridimensional reconstruction, the C-arm is moved through an angle of 180° and an X-ray image is recorded for a set of angular positions of the imaging assembly.

A 3D reconstruction is widely used during neurological examinations like the so-called aneurysm coiling and arterioveinous malformation (AVM) treatment to have a tridimensional reconstruction of the patient anatomy.

A 3D reconstruction also has a great clinical interest for other procedures like abdominal procedures, in which the region of interest lies in the abdominal region.

In such a case, the rotational acquisition is difficult to perform since there is a high risk that either the X-ray source or the X-ray detector knocks against the examination table. As a matter of fact, basically, the tunnel formed during the rotational movement of the imaging assembly has a rayon of 60 cm centered at the isocenter of the imaging assembly.

In addition, with conventional C-arm imaging apparatuses, the tridimensional field of view is limited. As a matter of fact, with a C-arm imaging apparatus having a conventional design, in which the distance between the X-ray source and the object to be imaged is of 72 cm, a distance between the X-ray source and the X-ray detector of 120 cm and a detector dimension of 40 cm, the field of view of imaging assembly is a cylinder having a diameter of around 20 cm, such that a significant region of an organ to be imaged may lie outside the area irradiated by the X-rays.

In the state of the art, several types of X-ray apparatuses have been developed to avoid the risks of having a collision between the imaging assembly and the examination table.

Reference can be made to documents US 2009/00 74 135 and US 7,684, 542.

The medical imaging systems disclosed in those documents proposes to avoid a table collision by dynamically modifying the isocenter position of the imaging assembly, usually formed by the rotation center of the rotary arm, during image acquisition.

In particular, positions of the X-ray detector are calculated to be tangential to an elliptical envelope surrounding the body of the patient, and a sequence of positions for the X-ray source is determined for each position of the X-ray detector.

In addition, selection can be made to determine whether a high resolution is desired or whether the largest possible volume of the region of interest is to be mapped to select a suitable position for the X-ray source.

However, with such imaging systems, it has been noted that the isocenter position of the imaging assembly is not fixed, such that the tridimensional region projected into the image plane is not constant. In other words, the portion of the organ imaged during the 3D reconstruction is a function of the C-arm position, degrading the image quality of the reconstructed 3D image.

In view of the foregoing, there is therefore a need to have a tridimensional X-ray imaging apparatus capable to avoid the risk that the X-ray source or the X-ray detector knocks against the examination table, while avoiding artefacts in the reconstructed tridimensionally means.

Another object of the invention is to provide a tridimensional X-ray imaging apparatus permitting to dynamically move the X-ray source to avoid a table collision, while keeping fixed the isocenter of the imaging assembly.

The subject matter of the invention is therefore, according to a first aspect, an X-ray imaging apparatus a rotary arm and an imaging assembly mounted on the rotary arm and comprising an X-ray source and an X-ray detector so that the X-rays emitted by the X-ray source are incidental to the detector.

According to a general feature of this apparatus, the X-ray source is coupled to the rotary arm by means of a movable support suitable to set the distance between the X-ray source and the object (SOD). In addition, the movement of the X-ray source is dynamically controlled during a tridimensional imaging.

By virtue of the mounting of the X-ray source on the rotary arm using a movable support, and the setting of the distance between the X-ray source and the object to be imaged, it is possible to move the image assembly along a non-circular path, avoiding all risks of collision with the examination table.

In addition, it has been noted that the size of the region of interest to be imaged can be increased.

Besides, the organ to be examined can be centered within the X-rays emitted by the source and detected by the X-ray detector to have a full reconstruction of the organ. In other words, contrary to conventional X-ray imaging apparatuses, in which the body of the patient must be centered to avoid table collision, the position of the examination table can be set in order to center the organ to be imaged.

According to another feature of the invention, the mobile support is adapted to move the X-ray source along the X-ray emission direction.

The movable support may comprise a telescopic support.

According to a further feature of the X-ray imaging apparatus, control means are provided to dynamically control movement of the X-ray source during the tridimensional imaging as a function of the rotation of the rotary arm.

The rotary arm may be coupled to a base assembly through a second movable support, suitable for extending and retracting the imaging assembly from the base assembly.

The X-ray imaging apparatus may further comprise an examination table having variable height.

Another subject of the invention, according to a second aspect, is a method for operating a X-ray imaging apparatus having a rotary arm and an imaging assembly mounted on said rotary arm and having a X-ray source and a X-ray detector, said X-ray source being coupled to the rotary arm by means of a movable support.

The position of the X-ray source is modified to set the distance between the X-ray source and the object to be imaged.

In addition, the movement of the X-ray source is dynamically controlled during a tridimensional imaging.

In one embodiment, the rotary arm is coupled to a base assembly by means of a second movable support, the imaging assembly being extended and retracted from the base assembly.

This method may further comprise the steps of adjusting the distance between the X-ray source and the object to be imaged and the distance between the X-ray source and the X-ray detector before image acquisition.

According to a further feature, the method may comprise the steps of:
- lifting the rotary arm to extend the imaging assembly from the base assembly;
- increasing the distance between the X-ray source and the object (SOD); and
- adjusting the position of the examination table according to the isocenter position of the imaging assembly.

Preferably, said steps of lifting the rotary arm, increasing the distance between the X-ray source and the X-ray detector, and adjusting the position of the examination table are carried out automatically.

Other objects, features and advantages of the invention will appear on reading the following description, given only as a non limited example, and made with reference to the appended drawings in which:
- figure 1 is a perspective view of an X-ray imaging apparatus according to one embodiment of the invention;
- figure 2 is a schematic view showing the path of the X-ray source during image acquisition; and
- figure 3 is a schematic view of the X-ray imaging apparatus according to the invention, showing the steps of adjusting the source to object distance (SOD) Reference is first made to figure 1, illustrating a perspective view of an X-ray imaging apparatus 1 for 3D medical imaging according to an embodiment of the invention.

This imaging apparatus 1 is in particular intended to generate an image sequence for a tridimensional reconstruction of an object to be studied, for example a part of the patient body. In one exemplary by non limited application of the invention, the object to be viewed is a region of interest of an abdominal organ.

As it can be seen, the imaging apparatus 1 includes an imaging assembly 2 operable to generate medical images of a human object lying on an examination table (not shown).

Imaging assembly 2 may include any suitable imaging means. In the illustrated embodiment, imaging assembly comprises an X-ray source, namely a X-ray tube 3, operable to generate X-rays in an emission direction and a X-ray detector 4 operable to generate a medical image of the subject.

The X-ray tube 3 and the X-ray detector 4 are placed at two mutually opposite ends of an arm 5, namely a C-arm supporting the imaging assembly, such that the X-rays emitted by the tube 3 are incidental to the detector 4.

As seen, the C-arm 5 has a front opening 5a for accommodating the subject being imaged, a lower distal end 5b supporting the X-ray tube 3, an upper distal end 5c supporting a X-ray detector and a curved back segment 5d having the arch shape.

The C-arm is slidingly mounted on a capture unit 6 coupled in a rotatable relation to an arm 7 by a rotation knuckle (not shown). The arm 7 is in addition mounted on a movable base assembly 8.

Therefore, the arm 7, the capture unit 6 and the C-arm 5 are all articulated relative to one another about articulation axes, so that the X-ray apparatus can be moved in three dimensions, and thus take images of an organ to be examined at various angles of incidence.

It should be noted that the base assembly 8 constitutes a mobile device provided with a running system comprising, for example, two lateral drives and steering wheels 8a placed at the rear, two free front wheels 8b, and means for driving the drive wheels comprising a steering motor coupled to a drive motor. The base assembly can thus be a robotised programmable device and may be associated with a navigation system capable, for example, of communicating by radio electric links with identification devices (not shown) placed in the operating room in order to allow the imaging apparatus 1 to locate itself precisely in the room and, in particular, relative to the examination table.

During radiography, the X-ray tube 3 and the X-ray detector 4 are brought to face a region of interest (ROI) in the body of a patient laid out on the examination table so that, when the region of interest is interposed between the X-ray tube 3 and the detector 4, it is irradiated by the X-rays, and the detector 4 produces representative data of features of the interposed region of interest.

In addition, during examination, the C-arm 5 supporting the X-ray tube and the X-ray detector is moved along an acquisition path through an angle of 180° around a rotation center to obtain X-ray images for different angular positions of the imaging assembly such that a 3D reconstruction can be achieved.

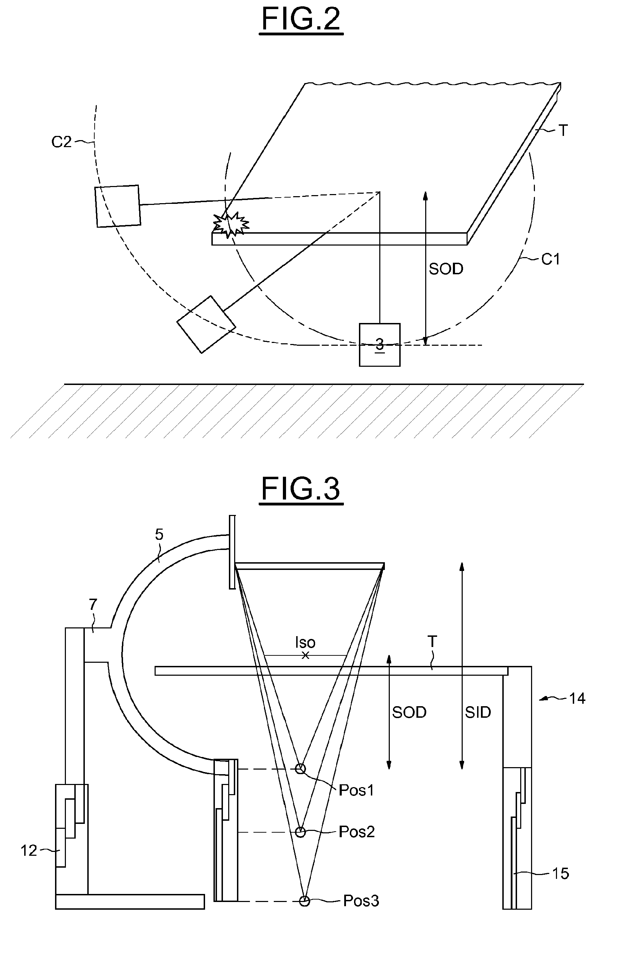

According to a first aspect of the X-ray imaging apparatus, and referring also to figure 2, the X-ray tube 3 is mounted on the lower distal end 5b of the C-arm 5 by means of a movable support 9 such that the X-ray tube 3 can be extended or retracted along the X-ray emission direction D (arrow A1).

In other words, and on the contrary to the X-ray imaging apparatuses in which the X-ray tube is moved along a circular part C1, such that, during a tridimensional image acquisition, the X-ray tube may knock against the examination table T, the movable support 9 is operable to increase the distance SOD between the X-ray source and the object to be imaged, before the tube 3 reaches the plane of the table to avoid collision therewith.

In other words, the movable support 9 is thus operable to move the X-ray tube 3 along non-circular trajectory C2.

The movable support 9 is coupled between the lower distal end 5b and the X-ray tube 3, for supporting the tube 3 and for extending and retracting this tube 3 according to the angular position of the C-arm 5. It is understood that the movable support 9 can include different arrangements and may comprise a telescopic support, for example a lift column, having one end embedded within the lower distal end 5b of the C-arm and an opposite end supporting the X-ray tube 3.

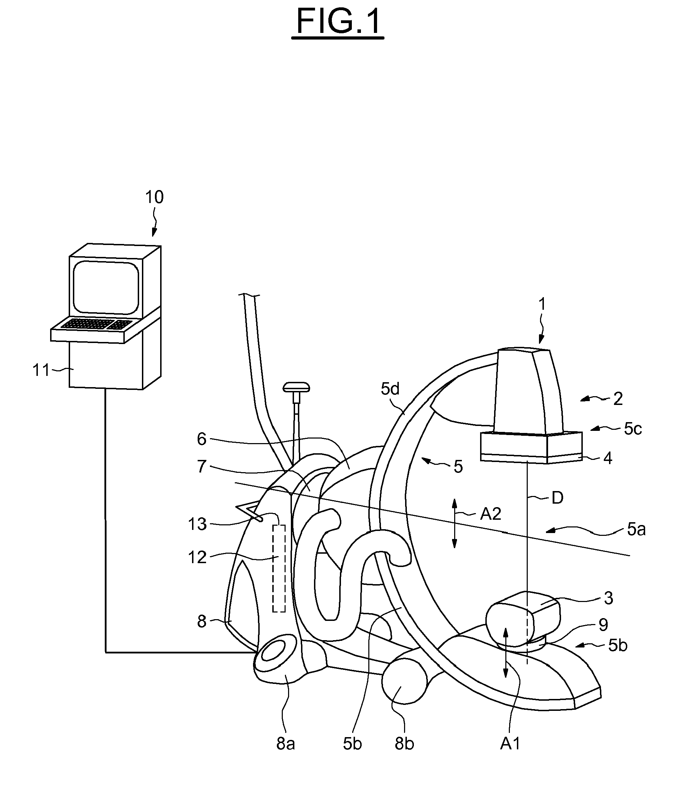

As further illustrated in figure 1, the X-ray imaging apparatus 1 is further provided with a central processing unit 10, schematically represented, that is provided with a control console 11, and is duly programmed to control the movement of the imaging assembly depending on the phases of an examination to be carried on.

In particular, the central processing unit 10 is furnished with storage means, of data storage memory type, for example of the ROM, RAM, etc.... type, incorporating one or more control algorithms capable of moving the movable base assembly 8 and the imaging assembly relating to the base assembly, in particular the rotation of the C-arm as well as the movement of the movable support 9, either automatically, or under the control of the control console 10, in response to instructions entered manually by an operator.

However, as concerns extending and retracting the X-ray tube 3 from the lower distal end 5b of the C-arm, this movement is preferably controlled automatically as a function of the rotational angle of the C-arm. It should be understood that information concerning the pivot angle of the C-arm may be obtained by different means, for example using sensors means.

It should be noted that the movable support used to set the distance between the X-ray source and the object to be imaged avoids tube collision when a tridimensional image acquisition is performed, in particular on an abdominal structure. In addition, surgeons will have the possibility to center the region of interest of an organ to be examined and have a full reconstruction of the organ.

Centering the region of interest of the organ on the isocenter of the imaging assembly thus permits to have a full reconstruction of the organ.

Besides, according to another aspect, the X-ray image apparatus 1 is further provided with a second movable support 12 embedded within the base assembly 8.

This second movable support is also controlled by the central processing unit 10 and is operable for extending and retracting the imaging assembly from the base assembly (Arrow A2).

It is to be understood that this second movable support can also be of different constructions. For example, in one embodiment, it comprises a lift column extending in the vertical direction within the base assembly and having an upper end 13 coupled to the arm 7 supporting the C-arm 5.

Referring to figure 3, the examination table is also mounted on a support 14 having a variable height and therefore provided with a lift column 15. Lift column 15 is also operable and controlled by the control processing unit 10 to set the height of the table according to the ongoing phase of an examination to be carried out.

In particular, the distance SOD between the X-ray source and the object to be imaged, and the distance SID between the source and the X-ray detector are adjusted for the 3D image acquisition.

Specifically, to have the maximum source to image distance SID and consequently have an increased tridimensional region of interest, before 3D image acquisition, the second movable support 12 is operated to lift the C-arm 5 and the imaging assembly supported thereby, and X-ray tube is lowered by the movable support 9 from position *Pos*1 to position *Pos*3. This movement increases the distance SOD.

The lift column 15 of the table support 14 is then operated to adjust the table height such that the object to be imaged reaches the isocenter position *Iso*. The risks of collision between the imaging assembly and the table are thus avoided, and the size of the reconstructed 3D region of interest is improved.

It should be noted that the above steps of operation are advantageously carried out automatically under the control of the control processing unit 10.

It has been noted that for different positions *Pos*1, *Pos*2*,* and *Pos*3 of the X-ray tube, the following SOD and SID distances and the following size for the region of interest ROI can be obtained:
Position *Pos*1 (without lift for the X-ray tube):
   SID = 120 cm
   SOD = 72 cm
   SID/SOD = 1,6
   ROI = 25 cm
Position *Pos*2 (with a lift of 50 cm for the X-ray tube):
   SID = 170 cm
   SOD = 122 cm
   SID/SOD = 1,39
   ROI = 28cm
Position *Pos*3 (with a lift of 100 cm for the X-ray tube):
   SID = 220 cm
   SOD = 172 cm
   SID/SOD = 1,27
   ROI = 31 cm.

## Claims

1. X-ray imaging apparatus, comprising a rotary arm (5) and an image assembly (2) mounted on the rotary arm and comprising a X-ray source (3) and a X-ray detector (4) so that the X-rays emitted by the X-ray source are incidental to the detector, **characterized in that** the X-ray source (3) is coupled to the rotary arm (5) by means of a movable support (9) able to set the distance between the X-ray source and the object, the movement of the X-ray source being dynamically controlled during a tridimensional imaging.

2. X-ray imaging apparatus according to claim 1, wherein the movable support (9) is adapted to move the X-ray source along the X-ray emission direction (D).

3. X-ray imaging apparatus according to claim 1 or 2, wherein the movable support comprises a telescopic support.

4. X-ray imaging apparatus according to any of claims 1 to 3, comprising control means (10) to dynamically control movement of the X-ray source during the tridimensional imaging as a function of the rotation of the rotary arm (5).

5. X-ray imaging apparatus according to any of claims 1 to 4, wherein the rotary arm (5) is coupled to a base assembly (8) through a second movable support (12) suitable for extending and retracting the imaging assembly from the base assembly.

6. X-ray imaging apparatus according to any of claims 1 to 5, comprising in addition an examination table (T) having a variable height.

7. Method for operating a X-ray imaging apparatus having a rotary arm (5) and an imaging assembly (2) mounted on said rotary arm having a X-ray source (3) and a X-ray detector (4), said X-ray source being coupled to the rotary arm by means of a movable support (9), **characterized in that** the position of the X-ray source is modified to set the distance between the X-ray source and an object to be imaged, the movement of the X-ray source being dynamically controlled during a tridimensional imaging.

8. Method according to claim 7, wherein the rotary arm (5) is coupled to a base assembly by means of a second movable support (12), the imaging assembly being extended and retracted from the base assembly.

9. Method according to claim 7 or 8, comprising the steps of adjusting the distance (SOD) between the X-ray source and the object to be imaged and the distance (SID) between the X-ray source and the X-ray detector before image acquisition.

10. Method according to claim 9, comprising the steps of:
- lifting the rotary arm (5) to extend the imaging assembly from the base assembly (8);
- increasing the distance between the X-ray source and the object; and
- adjusting the position of an examination table (T) according to the isocenter position of the imaging assembly.

11. Method according to claim 10, wherein said steps of lifting the rotary arm (5), increasing the distance between the X-ray source and the X-ray detector, and adjusting the position of the table are carried out automatically.
